Europäisches Patentamt

European Patent Office

Office européen des brevets

(11) Publication number: **0 186 125 B1**

## EUROPEAN PATENT SPECIFICATION

(45) Date of publication of patent specification: **10.07.91**

(51) Int. Cl.5: **C12N 11/08**, C12M 1/40, //C02F3/10

(21) Application number: **85116179.4**

(22) Date of filing: **18.12.85**

(54) **A carrier for microorganisms.**

(30) Priority: **24.12.84 JP 270719/84**

(43) Date of publication of application:
**02.07.86 Bulletin 86/27**

(45) Publication of the grant of the patent:
**10.07.91 Bulletin 91/28**

(84) Designated Contracting States:
**DE FR GB NL**

(56) References cited:
**US-A- 4 326 009**

**PATENT ABSTRACTS OF JAPAN, vol. 9, no. 271 (C-311)[1994], 29th October 1985; & JP-A-60 120 988 (ORGANO K.K.) 28-06-1985**

(73) Proprietor: **Chiyoda Chemical Engineering & Construction Company Limited**
**12-1, 2-chome Tsurumi-cho Tsurumi-ku**
**Yokohama-shi Kanagawa-ken(JP)**

(72) Inventor: **Noguchi, Masaaki**
**20-3, 2-chome Nakahara**
**Mitaka-shi Tokyo(JP)**
Inventor: **Yushina, Yoshinori**
**18-4, 2-chome Izumi-cho**
**Houya-shi Tokyo(JP)**
Inventor: **Sato, Hiromi**
**308-105, 2-chome Honmoku-cho Naka-ku**
**Yokohama-shi Kanagawa-ken(JP)**
Inventor: **Masuhara, Isao**
**No. 325, Shinsaku Takatsu-ku**
**Kawasaki-shi Kanagawa-ken(JP)**

(74) Representative: **Türk, Dietmar, Dr. rer. nat. et al**
**Türk, Gille + Hrabal Patentanwälte Brucknerstrasse 20**
**W-4000 Düsseldorf 13(DE)**

EP 0 186 125 B1

## Description

BACKGROUND OF THE INVENTION

The present invention relates to a carrier for microorganisms or, more particularly, to a carrier capable of supporting microorganisms in a high concentration in various types of so-called bioreactors used in waste water treatment, fermentative production and other microbiological processes.

Microbiological processes can be carried out as a continuous process by use of a bioreactor in which the requisite microorganisms are immobilized as being supported on a carrier. Various attempts and proposals have been made not only on the method for the immobilization of the microorganisms on a carrier but also on the material of the carrier per se for the effective immobilization of the respective microorganisms.

Needless to say, the role of the carrier for the immobilization of a microorganism is very important in order that a bioreactor can be operated with a high efficiency and good stability along with durability. What are required for a carrier of microorganisms include, firstly, a high concentration of the microorganism supported on the carrier, secondly, a high activity of the microorganism supported on the carrier and pertaining to the desired microbiological reaction and, thirdly, a long durability of the activity of the microorganism immobilized on the carrier to give a possibility of long-term continuous running of the bioreactor.

A method of entrapping microorganisms by the use of reagents such as calcium alginate, etc. is known as an immobilization of microorganisms. This method, however, has disadvantages that production of carrier, especially a lot of carrier is troublesome, the carrier resists to diffuse, the carrier is broken easily, the cost is expensive, etc.

Accordingly, in the present invention, the immobilization of microorganisms is performed by attaching (or depositing or adhering) the microorganisms to the carrier, though this method has a problem that some kinds of microorganisms are hardly to attach to the carrier.

There are known several inorganic particulate materials occurring in nature as a carrier capable of adhering microorganisms in a relatively high concentration including, for example, Shirasu balloons, pearlites and the like. Such inorganic particulate materials occurring in nature, however, are generally defective as a carrier of microorganisms because the particles thereof are fragile and readily absorb water to cause a change in the apparent specific gravity.

In view of the above mentioned problems in the natural inorganic materials, it is practiced to use solid, i.e. non-hollow, particles shaped of a foamed synthetic resin such as polyethylene, polypropylene, polystyrene and the like as a carrier of microorganisms. These solid particles of a foamed synthetic resin are disadvantageous due to the expensiveness although the specific gravity thereof is relatively stable. Therefore, it is also proposed to use cylindrical hollow particles of a synthetic resin.

A problem in the carrier particles shaped of a synthetic resin as such as is mentioned above is the low strength of adhesion between the microbial film and the surface of the particles so that the microbial film on the particle surface may sometimes be peeled off by the rubbing action between particles or between a particle and the reactor walls and by the shearing force caused between particles and the aqueous medium or air bubbles.

As a means for improving the adhesion of microorganisms, the inventors have previously proposed tubular particles as a carrier for supporting microorganisms (see Japanese Patent Kokai 58-198288) of which each particle is imparted with a roughened outer surface after abrasive scraping by a mechanical means.

As is readily understood, it is a preferable condition from the economical standpoint that the carrier material of the synthetic resin-made particles should have a weight per unit bulk volume expressed, for example, in $kg/m^3$ as small as possible to decrease the cost for the resin particles. In the tubular carrier particles having roughened outer surfaces, the value of the specific bulk weight in $kg/m^3$ can necessarily be not so small because the particles must have a strength to withstand the mechanical treatment in order to be imparted with roughness on the outer surface. Therefore, decreasing of the specific bulk weight in $kg/m^3$ is a contradictory requirement for carrier particles of this type having fully roughened surfaces of the tubular particles. The tubular carrier particles of weakly roughened surfaces having a decreased specific bulk weight in $kg/m^3$ are not suitable as a carrier for attaching (or adhering or supporting) microorganisms of a low growth rate or microbial cells having no adhesiveness or having no tendency for coagulation due to the unduly long time taken for the attachment thereof on the surface of the carrier particles. It is also a difficult matter to support a microorganism in a high density on such a carrier.

As is known, a bioreactor filled with a carrier attaching microorganisms on the surface is generally

2

operated as a fixed bed, fluidized bed or suspended bed. It is important in any of the bed types that the microorganism can readily attach on the surface of the carrier and that the microbial film formed on the carrier surface can always maintain a necessary thickness even after peeling or removal of the excessive amount of the microorganism due to intercontact of particles while the microbial film of a carrier of weakly roughened particles is sometimes completely lost by peeling when the carrier supporting the microorganism is used in a fluidized bed.

## SUMMARY OF THE INVENTION

It is therefore an object of the present invention to provide a carrier material for attaching microorganisms and usable in a bioreactor, which is free from the above described problems and disadvantages in the conventional carrier of prior art and can satisfy the various requirements for a carrier mentioned above.

Another object of the invention is to provide a carrier material for attaching microorganisms which is outstandingly inexpensive and more advantageous from the economical standpoint than the conventional ones along with the high performance.

Thus, the carrier material for attaching microorganisms provided by the present invention is a body in a cylindrical, tubular, angular or spherical form of which the diameter and the axial length are each in the range from 3 to 100 mm having a bulk density in the range from 0.005 to 0.4 g/cm$^3$ and a void ratio in the range from 60 to 95% formed by sterically intertwisted filaments having a diameter in the range from 10 to 1000 $\mu$m to comprise three-dimensionally intercommunicating spaces.

## BRIEF DESCRIPTION OF THE DRAWING

FIGURES 1a, 1b and 1c each illustrate a method for winding a covering material around the surface of a carrier body.

FIGURES 2a and 2b are an elevational front view and a plan view, respectively, of the apparatus used in Example 1.

FIGURE 3 is a graphic showing of the amount of the microorganism attached on a particle as a function of time in Example 1.

FIGURES 4, 5 and 6 are each a schematic illustration of the apparatus used in Example 2, Example 3 and Example 4, respectively.

FIGURE 7 is a schematic illustration of the apparatus used in Example 4 for comparative purpose.

FIGURE 8 is a schematic illustration of the apparatus used in the down-flow experiments in Example 5.

FIGURE 9 is a schematic illustration of the apparatus used in the up-flow experiments in Example 5.

FIGURES 10 and 11 are each a graphic showing of the relationship between the space velocity and the expansion rate of the fluidized bed representing the condition of fluidization of the carrier in Example 5 and Example 6, respectively.

## DETAILED DESCRIPTION OF THE PREFERRED EMBODIMENTS

As is mentioned in the above given summarizing description, the inventive carrier material for microorganisms is a body formed of filaments intertwisted sterically, i.e. in all directions. The intertwisted filaments produce complicated three-dimensional spaces in the carrier body and the microorganism is supported (or attached) on the surface of the filaments as well as in these three-dimensional spaces. The intertwisted filaments are bonded to each other at a part or all of the contacting points therebetween. The method for forming such a bonding between the filaments is not particularly limitative including mechanical means, thermal means and chemical means using an adhesive as well as combinations of these means. As a result of intertwisting of and bonding between the filaments, three-dimensionally intercommunicating spaces are obtained in the carrier body formed of the filaments so that the carrier body can exhibit larger and more advantegeous effect for the deposition and attachment of microbial cells. Moreover, the intercommunicating spaces are also available for the habitation of the microorganisms.

The diameter of the filaments to be intertwisted is not particularly limitative provided that no inconvenience is caused in the intertwisting thereof. However, it should usually be in the range from 10 to 1000 $\mu$m depending on the particular object of use of the carrier.

In respect of the material of the filaments, various kinds of organic and inorganic materials can be used in the form of filaments including synthetic resins such as nylon, polyester, acrylic resins, polyvinyl chloride, polyvinyl alcohol, polypropylene, polyurethane, polyvinylidene chloride and the like as well as cotton, rayon, cellulose acetate, glass and others.

3

It is necessary to sterilize a carrier being used for a bioreactor of fermentative production. As the material of the carrier which is subjected to a ordinary steam sterilization, such heat resistant resin as fluorine contained resin, silicone resin, etc can be used preferably. These materials are used in the form of monofilaments, yarns and rovings, woven and non-woven cloths, felts and so on.

Though not particularly limitative, the carrier material for microorganisms of the invention is used usually in a particulate form for the reasons that the form is advantageous by the lightness and suitable for mass production in a continuous process with low costs. When the carrier is used in a particulate form, the form should be cylindrical, tubular, angular or spherical though not particularly limitative thereto. In respect of the dimensions of such a cylindrical, tubular, angular or spherical particle, each of the diameter and, when the particle is cylindrical or tubular, axial length should be in the range from 3 to 100 mm or, preferably, from 5 to 50 mm. The bulk density of the particle should be in the range from 0.005 to 0.4 $g/cm^3$ or, preferably, from 0.005 to 0.2 $g/cm^3$. Further, the particle should preferably have a void ratio in the range from 60 to 95%.

If desired, the bulk density of the inventive carrier particles may be modified, i.e. increased or decreased. For example, the synthetic resin for forming the filaments is blended with a suitable amount of an inorganic or organic filler, such as calcium carbonate, talc and clay, before shaping into filaments so as to impart an increased bulk density to the carrier particles. On the other hand, the synthetic resin may be foamed to decrease the bulk density. It is sometimes advantageous to have fine particles of the above mentioned inorganic fillers, sand, abrasive powders and the like bonded to the surface of the filaments by adhesion. These adhesively bonded inorganic particles have an effect to increase the hydrophilicity of the carrier particles and acceptability of the filament surface to the attachment of microbial cells.

The inventive carrier for microorganisms is suitable for attaching various kinds of microorganisms including bacteria, yeasts, fungi, actinomyces, basidiomycetes and algae. The conditions for culturing the microorganisms using the inventive carrier can be conventional without particular limitations including aerobic and anaerobic conditions according to the particular object of the process for the production of useful products, waste water treatment, and others. Furthermore, the inventive carrier is also applicable to a mixed microbial system using two kinds or more of microorganisms so as to perform the waste water treatment or fermentative production under aerobic or anaerobic conditions.

The specific gravity of the filaments forming the inventive carrier for microorganisms should be determined according to the type of the bioreactor filled with the carrier supporting the microorganisms. For example, a carrier having a specific gravity smaller than 1 is suitable for filling a bioreactor of the down-flow fluidized bed or fixed bed type while a carrier having a specific gravity larger than 1 is used in a bioreactor of the up-flow fluidized bed or fixed bed type. A suspended-type bioreactor should be filled with a carrier having a specific gravity of 1 or in the vicinity of 1, e.g. in the range from 0.9 to 1.2.

Following is a comparative analysis of the inventive and conventional carriers for microorganisms made with an object to more fully distinguish the characteristics of the inventive carrier from the conventional.

To give an analysis of conventional carriers for microorganisms, following characteristics are in common in all of them. Namely, the object of the conventional carriers for microorganisms is to have the microbial cells attached on the surface of the carrier regardless of the type of the carrier including crushed stones for trickling filter, plastic-made plate packing for high rate trickling filter, plastic-made Raschig rings for high rate trickling filter, plastic-made discs used in the rotating disc filter, plastic-made honeycomb-shaped filtering materials for the contacting oxidation tank, tubular particles having roughened outer surfaces proposed by the inventors, sands and active charcoals used in fluidized bed reactors and the like. The basic principle common in these conventional carriers is the planar attachment of microbial cells on the surface of the carrier cells. This principle of planar attachment of the microbial cells on the surface of the carrier cells originates in the aerobic waste water treatment and a problem in this case consists in the thickness of the film formed by the attachment of the microbial cells in relation to the diffusion of the dissolved oxygen into the film. Since a microbial film having an excessively large thickness unavoidably includes a portion under an anaerobic condition, it has been generally pointed out that a microbial film on the carrier surface may have a thickness not exceeding a certain upper limit of, for example, 0.6 mm but no larger thickness has advantages.

On the other hand, the basic principle in the inventive carrier for microorganisms is that the microbial cells are not only attached on the surface of the filaments but also held three-dimensionally by utilizing the three-dimensional spaces formed by the intertwisted filaments constructing the body of the carrier. In addition, the three-dimensional spaces are intercommunicated with each other from any surface portion to the core portion of a carrier body having a complicated structure. Accordingly, the cylindrical, tubular, angular or spherical body of the carrier with such a structure has penetrability to water from a portion to any other portion of the surface. This feature is in great contrast to conventional carriers such as a plastic-made Rasching ring of a tubular form of which the outer and inner surfaces of the tube are not intercommunicated through the body although they each provide a bed for the attachment (or deposition) of the microbial cells.

When the inventive carrier for microorganisms having the above described features is used in a bioreactor, an extremely high efficiency is obtained in the processes of fermentative production and waste water treatment as is described in Examples given below.

It may be a possible point of view that, even in the conventional carriers for microorganisms, the microbial bodies are attached three-dimensionally at least when the microbial film has grown to have a substantial thickness. Such a build-up of the microbial film, however, is quite different in principle from the three-dimensional cumulation of the microbial cells in the inventive carrier since conventional carriers support the microbial cells merely two-dimensionally though with growth of the film thickness.

As viewed from an economical standpoint, the inventive carrier for microorganisms has an outstandingly small weight per unit bulk volume in comparison with conventional carriers to give a great economical advantage. For example, a carrier particle of the invention prepared of nylon filaments may have a specific bulk weight of as small as 40 kg/m$^3$ with a very large void ratio.

It is also noted that the inventive carrier body or particle may have a mechanical strength as high as desired by using filaments having an appropriate diameter.

The inventive carrier for microorganisms has a further advantage that the rate of attachment and growth of the microorganisms thereon is much larger than on the conventional carriers so that the bioreactor filled with the inventive carrier can enter the stage of proper running after a remarkably decreased number of days taken for the preparatory attachment and growth of the microorganisms thereon contributing to a great improvement in the productivity of the bioreactor plant.

Accordingly, the inventive carrier for microorganisms is particularly satisfactory as a carrier for attaching (or supporting) microorganisms of low multiplication rate such as nitrification and methane fermentation bacteria and microorganisms having poor adhesiveness to the substrate surface such as the yeasts used in the fermentative production and certain bacteria, e.g. micrococci and bacilli. Therefore, the inventive carrier for microorganisms is useful as a carrier to fill a bioreactor in the processes of fermentative production and in the waste water treatment by the activated sludge method with great advantages.

Since each particle of the inventive carrier for microorganisms is composed of a large number of intertwisted filaments, the particles themselves are entangled with each other by the intertwinement of the outer filaments as is observed in Example 4 described below. Although the intertwinement of the filaments per se has no particularly adverse influence on the microbial reaction, following disadvantages and inconveniences are caused thereby. Intertwinement of the filaments to cause entanglement of the carrier particles may give a limitation to the free selection of the type of the bioreactor including the fluidized bed, suspended bed and moving bed type ones. Entanglement of the carrier particles in a fixed-bed bioreactor may be inhibitive of the back-washing and air washing of the bed which should be mixed when the bed has been plugged by the outgrowing microorganisms. The inventors' investigations have led to a solution of this problem according to which the entanglement of the carrier particles by the intertwinement of the filaments can be dissolved by winding a covering material around each particle of the carrier. In the course of the development of such improved carrier particles, three kinds of covering materials were used in the tests as described below.

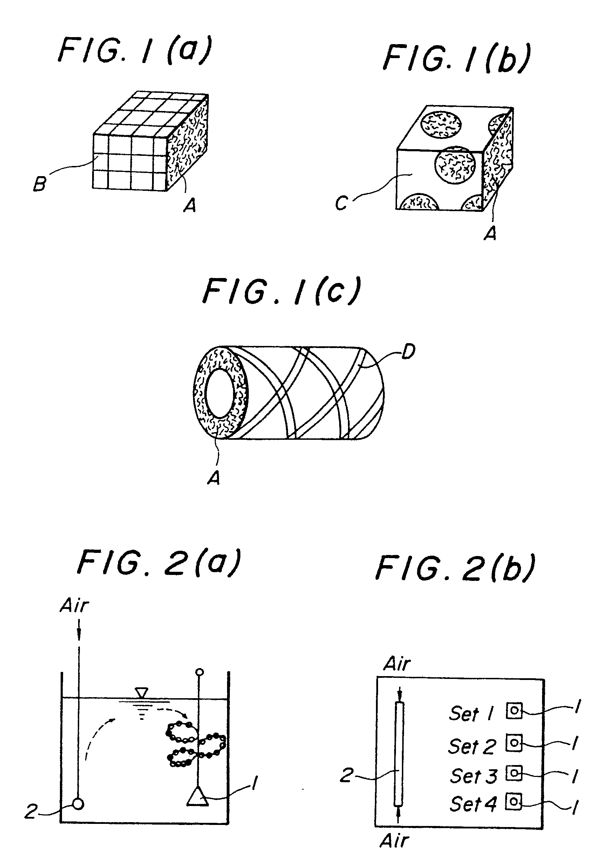

As is illustrated in FIGURE 1a, in the first place, a carrier particle A made of nylon filaments in a cubic form of about 10 mm by 10 mm by 10 mm dimensions was wrapped on four of the six facets with a net B of polyester filaments of 0.3 mm diameter in a plain lattice-wise network with a pitch of 3.5 mm.

Secondly, as is illustrated in FIGURE 1b, the same cubic carrier particle A as above was wrapped on four of the six facets with a perforated nylon film C having a thickness of 0.2 mm and provided with circular openings of 6 mm diameter in a zigzag disposition at a pitch of 10 mm.

Thirdly, as is illustrated in FIGURE 1c, the same nylon-made carrier body as used in the first test but having 65 mm by 27 mm by 8 mm dimensions was put into a net ring D of polypropylene having an outer diameter of 27 mm, inner diameter of 21 mm, length of 27 mm and weight of 2.5 g per piece so that the carrier body was compressed therein to take a cylindrical form.

In the first and second of these three tests, the covering material was bonded to the carrier body by use of a binding agent while, in the third of the tests, the covering material was bonded to the carrier body by sawing with a thread.

It is of course that a covering material can be bonded to the carrier body by any of the physical, chemical and thermal means according to need. What is important in the selection of the covering material is that the intertwinement of the filaments is decreased as far as possible while the exposed surface area of the carrier body should be as large as possible. It was found in each of the above described three covering tests that the thus covered carrier bodies can be fluidized each as an isolated piece without entanglement therebetween.

Secondarily, entanglement of the carrier bodies by the intertwinement of the respective filaments can be prevented or reduced also in the following manner. When the carrier bodies are in the form of particles, the surface of each particle in the course of fabrication is pressed at an elevated temperature to eliminate protrusion of the filaments out of the surface of the particle as completely as possible so as to smoothen the surface of the particle. For example, a sheet of a non-woven fabric made of a synthetic resin is moved on a hot plate heated by means of a built-in heater under compression with a similar hot plate on the fabric to have a desired thickness so that the non-woven fabric coming out of the hot plates is imparted with smoothened surfaces. The thus obtained carrier particles are freed from intertwinement of the respective filaments which otherwise causes entanglement of the particles so that they can be easily fluidized in a fluidized-bed bioreactor. Furthermore, compression at an elevated temperature has an effect to increase the rigidity of the carrier particle. Tough carrier particles can be prepared of a non-woven fabric of filaments in this manner.

While the inventive carrier body is formed of intertwisted filaments having a diameter in the range from 10 to 1000 $\mu$m as is stated before, it is not always necessary that all of the filaments have the same diameter but optional to use filaments having two or more different diameters. Rather, several advantages are obtained by use of filaments having different diameters that the resultant carrier body is a composite of a body composed of the coarser filaments and a body composed of the finer filaments having a more complicated structure than a carrier body composed of the filaments of the same diameter so that the capacity of the carrier body for the attachment and holding of the microbial cells is further increased and that the finer filaments can be melted earlier than the coarser filaments by heating contributing easier and more reliable bonding of the filaments.

Assuming that the diameters of the coarser and finer filaments are D $\mu$m and d $\mu$m, respectively, and they are used for the preparation of the inventive carrier bodies in amounts of W kg and w kg, respectively, it is preferable that the relationships
D/d = 2 to 10 and W/w = 0.25 to 100
are held between these parameters. When the value of D/d is 1 or larger but smaller than 2, no definite difference can be noted in the velocity of melting by heating between the coarser and finer filaments. When the value of D/d is larger than 10, on the other hand, the proportion occupied by the coarser filaments is decreased and the rigidity of the carrier body is decreased. When the value of W/w is smaller than 0.25, the rigidity of the carrier body is decreased, and W/w is larger than 100, the weight proportion of the finer filaments is so small that no particular advantage is obtained by the combined use of the coarser and finer filaments.

The carrier body for microorganisms according to the invention can also be shaped in a sheet-like form of 1 to 20 mm thickness which is used in combination with a plate-like or tubular reinforcement made of a board or net. For example, a sheet-like carrier body for microorganisms according to the invention can be used as bonded to the surface of plate-like filtering materials in a high rate trickling filter.

The covering material to be wound around the surface of the carrier body of the invention is usually made of a thermoplastic resin such as polyester, nylon, polypropylene and the like although there is no particular limitation thereto including various kinds of other materials. The materials of the covering and the filaments intertwisted into the carrier body may be the same or may be different from each other. The carrier body made of the intertwisted filaments and the covering material can be bonded together by any of physical, thermal and chemical adhesive bonding methods.

Although the covering material to be wound around the surface of the carrier body may be in the form of a net, perforated film, net ring and any other forms as is mentioned above, at least 30% or, preferably, at least 50% of the surface area of the carrier body should be exposed at any rate on the surface covered with the covering material.

In the following, examples are given to illustrate the carriers for microorganisms according to the invention in more detail.

EXAMPLE 1
—————— —

Three different carriers each in a particulate form I, II and III were used in the tests, of which the first two were according to the invention while the last one was conventional as disclosed in Japanese Patent Kokai 58-198288. Table 1 below gives the characterization of each of the carrier particles I, II and III having appearance described below.

Carrier particle I was made of complicatedly intertwisted filaments of 40 $\mu$m diameter and most of the contacting points between the filaments were bonded together. Further, fine particles of an inorganic abrasive powder were adhesively bonded to the surface of the filaments.

Carrier particle II was made of complicatedly intertwisted filaments of 40 $\mu$m diameter and shaped into a spherical form by gently heating the surface.

Carrier particle III in a tubular form had a roughened outer surface with irregularities and whiskers after scrubbing with a wire brush.

## Table 1

| Carrier particle | I | II | III |
|---|---|---|---|
| Configuration | Cylindrical | Spherical | Tubular |
| Outer diameter, mm | 5 | 7 | 4.5 |
| Length, mm | 5 | - | 4.5 |
| Wall thickness, mm | - | - | 0.3 |
| Bulk density, g/cm$^3$ | about 0.04 | about 0.07 | about 0.13 |
| Material | Nylon | Polypropylene | Polypropylene |

The above characterized three types of carrier particles were each used in the test for the deposition of microbial cells using an activated sludge aeration tank of 1 m by 1 m by 1 m dimensions with an effective capacity of about 1 m$^3$ having about 3000 mg/liter of MLSS as the experimental apparatus into which a simulated waste water containing glucose as the major contaminant to give a BOD of about 1000 mg/liter was continuously introduced at a rate of 300 liters/day at a temperature in the range from 15 to 20°C.

In the microbial attachment tests, four strings each made up with 10 pieces (carrier particles I and II) or 100 pieces (carrier particle III) stringed with a nylon thread and provided with a sinker 1 at the end as is illustrated in FIGURES 2a and 2b were dipped in the simulated waste water in the aeration tank and they were taken out of the tank one by one at 1 week intervals from the start of dipping to examine the amount of the deposited microbial cells. During the four weeks period, the simulated waste water in the tank was continuously aerated by means of the air sparger tube 2 shown in the figures.

The amount of the deposited microbial cells on the carrier particles I and II was determined in the following manner. Each of the carrier particles taken out of the aeration tank was first squeezed to press out the deposited microbial cells and then crumpled several times in water in a 300 ml beaker so as to wash the attached microbial cells out of the carrier particle repeatedly until the washing water in the beaker no longer became cloudy by the suspended microbial bodies assuming complete isolation of the microbial cells. Thereafter, the microbial cells in the first squeeze and those suspended in the washing water were respectively collected by filtration on a filter paper and dried to determine the dry weight of the microbial cells as a total.

In the test using the carrier particles III, the weight of the four strings before and after the test was determined after drying at 105°C for 4 hours and the difference between the two values was taken as the dry weight of the attached microbial cells.

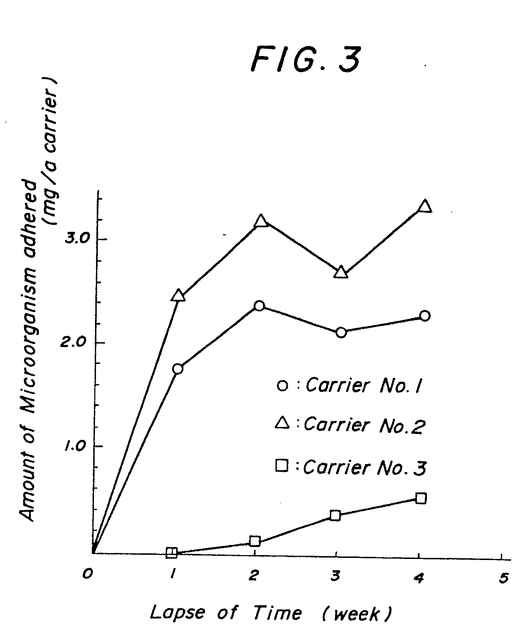

The results of the amounts of the attached microbial cells obtained after 1, 2, 3 and 4 weeks of dipping are shown in Table 2 below and FIGURE 3 of the accompanying drawing.

Table 2

| No. of pieces | Amount of deposited microbial bodies, mg | | |
| | Carrier particle I | Carrier particle II | Carrier particle III |
| Weeks of dipping | 10 pieces | 10 pieces | 100 pieces |
| 1 | 17.7 | 25.1 | almost 0 |
| 2 | 24.0 | 32.3 | 16.6 |
| 3 | 21.5 | 27.8 | 40.0 |
| 4 | 23.5 | 34.1 | 61.4 |

Table 2 and FIGURE 3 clearly indicate that the carrier particles for microorganisms according to the invention are much better than the conventional ones not only in the ultimate amount of the attached microbial cells but also in the velocity of deposition of the microbial bodies thereon.

EXAMPLE 2

An experiment of fermentative production of ethyl alcohol was undertaken with glucose as the substrate using carrier bodies according to the invention for filling a reactor.

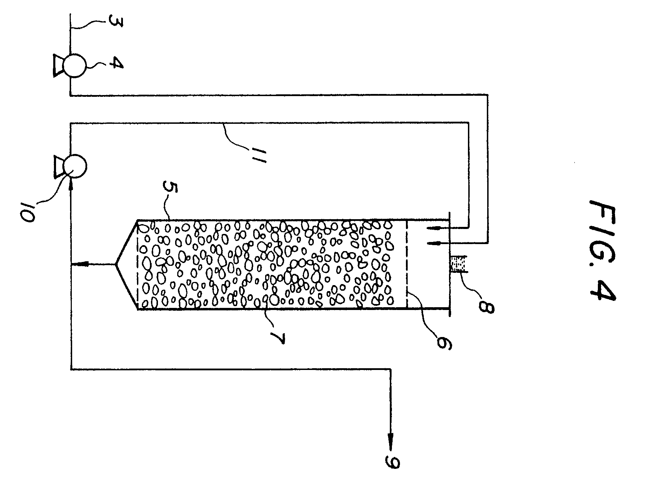

The experiment was carried out in a glass-made reactor column illustrated in FIGURE 4 having an inner diameter of 35 mm, height of 250 mm and capacity of 240 ml and filled with carrier particles to form a fixed bed of 240 ml volume. The experiment was started after a sterilization treatment of all parts including the reactor column, carrier particles, pipe lines for the feed of the culture medium and others.

Each of the carrier particles had a cylindrical configuration of about 5 mm diameter and about 5 mm axial length and was made of nylon filaments of about 40 $\mu$m diameter complicatedly intertwisted in all directions. Most of the contacting points between the nylon filaments were bonded together and fine particles of an inorganic abrasive powder were adhesively bonded to the surface of a part of the nylon filaments. Each of the carrier particles had a bulk density of about 0.075 g/cm$^3$.

The culture medium was an aqueous solution of glucose in a concentration of 15% by weight and the microorganism used for the fermentation was Zymomonas mobilis ATCC 10988. The experiment was performed as a continuous running for 1250 hours at a controlled temperature of 30° C.

The experimental procedure was as described below with reference to FIGURE 4. Thus, the culture medium was introduced into the distributor 6 in the experimental apparatus 5 from the feed line 3 through the pump 4. The culture medium flowed downwardly in the experimental apparatus 5 from the top to the bottom between and through the carrier particles 7 attaching the microorganism so that the glucose as the substrate was converted into ethyl alcohol by the microbiological reaction. The effluent liquid coming out of the bottom of the experimental apparatus 5 was mostly recycled through the recycling line 11 by means of the pump 10 while a portion corresponding to the influent culture medium was discharged out of the discharge pipe line 9. The volume of the recycling liquid was about 160 ml/minute corresponding to a space velocity of 10 meters/hour for the experimental apparatus 5. The carbon dioxide gas produced by the microbiological reaction was discharged out of the apparatus through the exhaust tube 8.

The results of the experiment when a stationary state had been reached were as follows.

Starting concentration of glucose: 14.1% by weight
Feed rate of culture medium: 200 ml/hour
Residual concentration of glucose: 1.2% by weight
Concentration of ethyl alcohol in effluent: 6.5% by weight
Yield of ethyl alcohol, per theoretical value: 90%
Rate of ethyl alcohol production: 65 g/liter filled volume/hour

As is understood from the above given results, the productivity was very high for the fermentative production of ethyl alcohol. The above given yield of ethyl alcohol and rate of ethyl alcohol production were calculated using the equations given below.

Yield of ethyl alcohol, % = [(concentration of ethyl alcohol in the effluent/concentration of glucose in the feed)] x (180/92) x 100

Rate of ethyl alcohol production, g/liter filled volume/hour = [(concentration of ethyl alcohol in the effluent/volume of filled volume, liter)] x (feed rate of ethyl alcohol)

EXAMPLE 3

An experiment of fermentative production of citric acid by a process of aerobic fermentation was undertaken with glucose as the substrate using carrier particles for microorganisms for filling a reactor. The conditions and procedure for the experiment were as follows.

Experimental conditions

Reactor: the reactor was a glass tube having an inner diameter of 50 mm and a height of 400 mm with a capacity of 785 ml and the culture medium therein was aerated by introducing pure oxygen at the bottom of the reactor column. A downward flow of the culture medium was formed by utilizing the discharged gas with a gas lift tube.

Number of stages: 1

Carrier particles: each of the carrier particles was in a spherical form of about 7 mm diameter formed of intertwisted polypropylene filaments, of which the diameter was in the range from 5 to 100 $\mu$m with an average of 40 $\mu$m, and had a bulk density of 0.17 g/cm$^3$.

Column filling: 300 ml as bulk volume of the carrier particles were introduced into the reactor column corresponding to about 40% of the column capacity.

Microorganism: Aspergillus niger IAM 2093

Substrate in the culture medium: glucose

Temperature: 30 °C

Experimental procedure

[1] Pre-culturing

A strain of Asp. niger IAM 2093 was cultured in a culture medium at a pH of 6.0 containing 10% by weight of glucose under aeration at 30 °C for 4 days with agitation together with the above mentioned carrier particles of the invention so as to attach the microbial cells on the carrier particles.

[2]

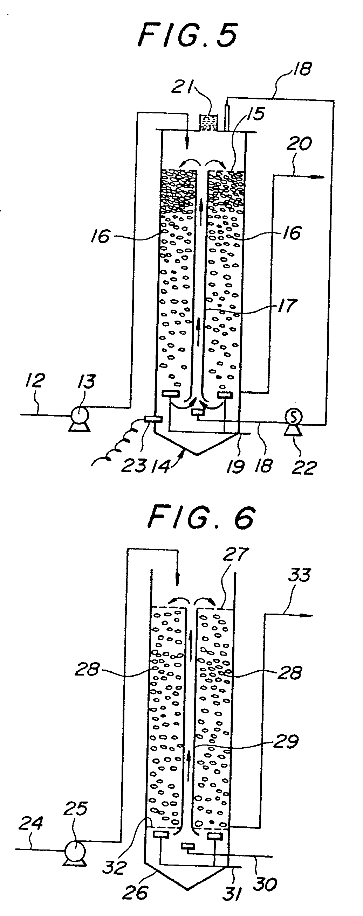

In the following, the experimental procedure is described with reference to FIGURE 5 of the accompanying drawing.

In the first place, the carrier particles 16 on which the microbial cells of Asp. niger had been deposited (or attached) as described in [1] above were introduced in the apparatus 14 for continuous experiment. A culture medium at a pH of 3.0 containing 10% by weight of glucose was introduced into the distributor 15 inside the experimental apparatus 14 through the feed line 12 by means of the pump 13. The culture medium flowed downwardly in the experimental apparatus from the top to the bottom between and through inside of the carrier particles 16 attaching the microbial cells of Asp. niger where glucose as the substrate was converted into citric acid by the microbiological reaction. The oxygen gas necessary for the aerobic reaction was supplied to the apparatus through the gas line 19 and, simultaneously, the exhaust gas still rich in the oxygen content was introduced into the bottom of the gas lift tube 17 through the gas circulation line 18 and the compressor 22 so as to form an upward flow in the gas lift tube 17 and a downward flow through the bed of the carrier particles 16. Any excess volume of the exhaust gas was discharged out of the exhaust tube 20. The culture medium after completion of the citric acid fermentation was taken out of the discharge tube 20 at the bottom of the experimental apparatus 14. The concentration of the dissolved oxygen in the culture medium was monitored by means of the sensor 23 so as to maintain the oxygen concentration at approximately 10 mg/liter by controlling the feed rate of the oxygen gas through the gas line 19.

Results of experiment

The experiment of citric acid fermentation carried out in the above described manner gave the results shown below at the stationary state.

Concentration of glucose in the feed: 10% by weight

Rate of feed: 35 ml/hour

Concentration of residual glucose: 0.3% by weight

Concentration of citric acid in the effluent: 6.1% by weight

Yield of citric acid: 61%

Rate of citric acid production: 7.12 g/liter/hour of the bulk volume of carrier particles or 2.72 g/liter/hour of the reactor volume

The above given yield of citric acid was calculated using the following equation.

Yield of citric acid, % = [(concentration of citric acid in effluent/concentration of glucose in culture medium)] x 100

## EXAMPLE 4

An experiment of aerobic treatment of waste water containing phenolic compounds was carried out using carrier bodies for microorganisms according to the present invention. For comparative purpose, an experiment by the activated sludge process was concurrently undertaken according to the procedure widely employed in the prior art. The waste water subjected to these experiments contained phenol, cresol and the like as the phenolic compounds. Followings are the conditions, procedure and results of the experiments.

### Experimental conditions

[1] Waste water treatment using the inventive carrier bodies

Reactor: a cylinder made of a transparent polyvinyl chloride resin having an effective diameter of 150 mm and a height of 1130 mm with a capacity of 20 liters

Number of stages: 1

Carrier particles: the same ones as used in Example 2 excepting the configuration which was a cube of 10 mm by 10 mm by 10 mm

Packing ratio: 50% with 10 liters of the carrier particles in bulk

Microorganism: activated sludge

[2] Waste water treatment by the comparative activated sludge process

Reactor: a rectangular tank of 150 mm by 140 mm wide and 236 mm depth with a capacity of 6 liters

Number of stages: 1

Carrier particles: none

Microorganism: about 3000 mg/liter of activated sludge

[3] Experimental conditions common in the two experiments

Waste water treatment:

COD = 400 mg/liter

phenols = 225 mg/liter

SS = almost 0 mg/liter

Temperature: 20°C

pH: neutral

Period of experiment: about 3 months

### Experimental procedure

[1] Waste water treatment using the inventive carrier bodies

The procedure is described below with reference to FIGURE 6 of the accompanying drawing. In the first place, the waste water containing the phenolic compounds was introduced into the distributor 27 in the reactor 26 through the feed line 24 by means of the pump 25. The waste water flowed downwardly in the reactor 26 between and through the carrier particles 28 supporting the activated sludge attached thereon where the phenolic compounds were decomposed by the microbiological reaction and converted into harmless water and carbon dioxide. The oxygen required for the aerobic reaction was introduced into the reactor 26 through the air feed line 31. Air was introduced into the air lift tube 29 at the bottom thereof through the air feed line 30 so as to form an upward flow in the air lift tube 29 and a downward flow in the bed of the above mentioned carrier particles 28 filling the reactor 26. A net 32 was provided at the bottom of the bed of the carrier particles to receive descending particles. Accordingly, the waste water was circulated between the air lift tube 29 and the bed of the carrier particles 28 and discharged out of the discharge tube 33. The concentration of dissolved oxygen in the waste water was continuously monitored and controlled so as to maintain a concentration of at least 1 mg/liter.

[2] Waste water treatment by the comparative activated sludge process

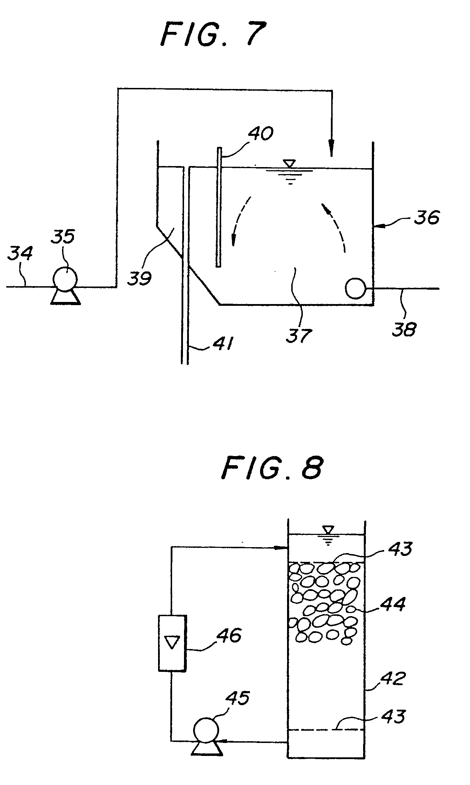

The procedure is described below with reference to FIGURE 7 of the accompanying drawing. The same waste water as above containing the phenolic compounds was introduced into the aeration tank 37 of the experimental apparatus for the activated sludge process 36 through the feed line 34 by means of the pump 35. The experimental apparatus for the activated sludge process 36 was partitioned into an aeration tank 37 and a settling tank 39 with a partition board 40. The sediment of the activated sludge on the bottom of the settling tank 39 flowed spontaneously into the aeration tank 37. The capacity of the reactor mentioned before means the volume of this aeration tank 37. The oxygen required for the aerobic reaction was supplied to the aeration tank 37 through the gas feed line 38 and the concentration of oxygen therein was maintained always to exceed 1 mg/liter. The waste water containing the activated sludge after aeration in the aeration tank 37 was transferred to the settling tank 39 where the activated sludge was separated from water by gravitational sedimentation and the thus treated water was discharged out of the discharge tube 41 to be discarded.

Results of experiment

The first one month during the continuous running for 3 months was spent for the acclimatization of the microorganisms to phenols and growth of the microorganisms. Following Table 3 gives various values obtained at a stationary state where the concentration of the phenolic compounds in the treated water was 1 mg/liter or lower.

## Table 3

|  | Inventive test | Comparative test |
|---|---|---|
| COD in the feed waste water, mg/liter | 400 | 400 |
| Phenols in the feed waste water, mg/liter | 225 | 225 |
| COD in the treated water, mg/liter | 12 | 9 |
| Phenols in the treated water, mg/liter | 0.33 | 0.46 |
| Rate of waste water feed, ml/hour | 3540 | 200 |
| Residence time in the reactor, hours | 5.6 | 30 |
| COD load, kg/m$^3$ tank·day | 1.7 | 0.32 |
| Phenol load, kg/m$^3$ tank·day | 0.96 | 0.18 |

In the comparative test by the activated sludge process, as is shown by the results given in Table 3, the maximum phenol load was 0.18 kg/m$^3$ tank·day when the desired concentration of the phenolic compounds in the treated water was 1 mg/liter or lower and the quality of the treated water rapidly decreased as the phenol load was increased over this value.

In the test running using the carrier particles according to the invention, on the other hand, the desired concentration of the phenolic compounds in the treated water of 1 mg/liter could easily be achieved even when the phenol load was as high as 0.96 kg/m$^3$ tank·day which was even more than 5 times larger than in the conventional activated sludge process. It was noted in this test using the carrier particles that the carrier particles were floating in the reactor at the beginning stage of the continuous running followed by subsequent settling. The carrier particles were observed to be in an entangled condition with each other.

11

EXAMPLE 5

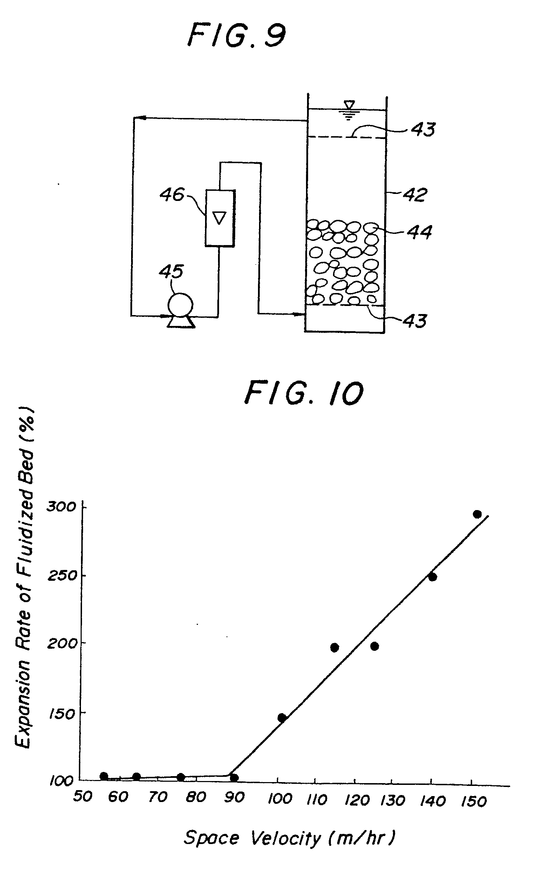

Experiments were undertaken for the fluidization of the carrier particles using the carrier particles of the invention with or without coverings thereon. Two different apparatuses were used in the experiments as illustrated in FIGURES 8 and 9 for a downward flow and upward flow, respectively. Details of the experiments were as follows.

Test column 42: a cylinder made of a transparent polyvinyl chloride resin having an effective diameter of 150 mm and a height of 1130 mm with a capacity of 20 liters

Capacity of circulation pump 45: 0.25 to 2.5 m³/hour

Carrier particles, each used in a bulk volume of 10 liters

[1] Carrier particles without coverings: the same nylon filament-made 10 mm by 10 mm by 10 mm cubes as used in Example 4

[2] Carrier particles with coverings: the same nylon filament-made cubes as in [1] above covered with a polyester net as illustrated in FIGURE 1a

[3] Carrier particles with coverings: the same nylon filament-made cubes as in [2] above covered with a perforated nylon film as illustrated in FIGURE 1b

[4] Carrier particles with coverings: cylindrical bodies of 27 mm outer diameter and 27 mm length made of nylon filaments and covered with a polypropylene-made net ring as illustrated in FIGURE 1c

The carrier particles of each of the above described 4 kinds were introduced into the test column and subjected to the fluidization test either in an upward flow, when they were sinkable in water, or in a downward flow, when they were floatable on the surface, to visually examine the state of fluidization. The flow rate of the circulating water was in the range from 0.25 to 2.5 m³/hour. The results are shown in Table 4 below. FIGURE 10 graphically shows the expansion rate of the above described carrier particles [4] as a function of the space velocity to give the fluidization characteristics of the carrier particles. Incidentally, the flow rates 0.25 m³/hour and 2.5 m³/hour of the circulating water correspond to the space velocities of 14 m/hour and 140 m/hour, respectively.

Results of experiment

Table 4

| Carrier particles | Direction of water flow | Experimental result, condition of fluidization |
|---|---|---|
| [1] | Upward | No fluidization due to entanglement |
| [2] | Upward | Good fluidization |
| [3] | Upward | Good fluidization |
| [4] | Downward | Good fluidization |

EXAMPLE 6

A further fluidization test was undertaken using the carrier bodies prepared in the following manner. A non-woven fabric of polypropylene fiber having a thickness of about 10 mm was spread flat on an iron hot plate kept at about 250° C by means of the built-in heater and gently pressed with an iron plate put thereon. Then, the non-woven fabric was reversed upside down and the other surface thereof was heated in the same manner under compression. The thus heat-treated non-woven fabric was shaped into tubular carrier bodies. Followings are several parameters of the tubular carrier bodies.

Material: polypropylene filaments having two different diameters

Filament diameters: about 110 $\mu$m (D) and about 34 $\mu$m (d)

Weight ratio of filaments having different diameters: 1:1

True density of material: 0.95 g/cm³

12

Dimensions of carrier body: outer diameter 35 mm, inner diameter 25 mm, wall thickness 5 mm and length 35 mm

Bulk density of carrier particle: 0.039 g/cm³

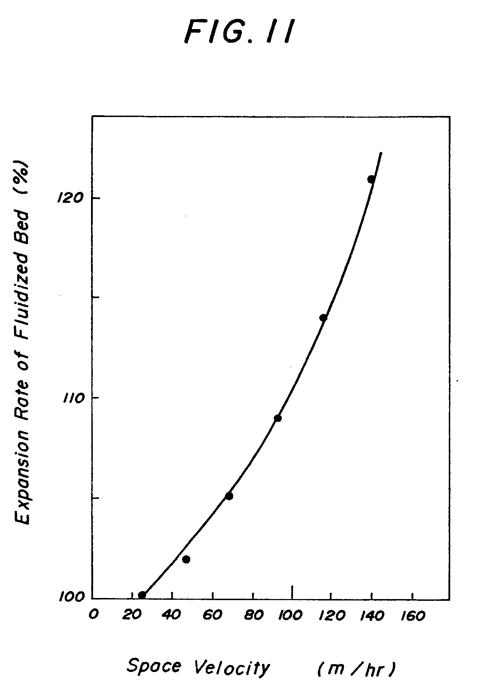

The carrier bodies were used in a fluidization test using the same apparatus of experiment as illustrated in FIGURE 8 and used in Example 5 with a downward flow of the circulating water. Thus, 10 liters of the carrier particles in bulk volume were introduced into the test column 42 and fluidized by the downward flow of the circulating water at a varied flow rate of 0.25 to 2.5 m³/hour to determine the expansion rate of the carrier bed as a function of the flow rate of water. The results were as shown in FIGURE 11, from which it is clear that the fluidized bed was duly expanded as the flow rate of water was increased with almost no entanglement between the carrier bodies.

EXAMPLE 7

The carrier bodies used in this Example were the same ones as used in Example 6 except that the non-woven fabric of polypropylene of which the carrier bodies were shaped was composed of a 10:1 by weight blend of the filaments of about 110 $\mu$m diameter and filaments of about 34 $\mu$m diameter. The characteristic parameters were approximately identical with those of the carrier bodies used in Example 6.

The apparatus used in the experiment was the same one as used in Example 5 and the cylindrical reactor was half-filled with the carrier bodies to occupy 50% of the capacity. A simulated waste water prepared by dissolving ammonium chloride in water in an amount to give a concentration of nitrogen of 500 mg/liter in the form of $NH_4$ with addition of small amounts of sodium hydrogencarbonate $NaHCO_3$ and potassium dihydrogenphosphate $KH_2PO_4$ was introduced into the reactor and inoculated with an activated sludge to start the experiment of nitrification under conditions of a pH of approximately 7.2 and temperature of 25°C. While the experiment was continuously run for about 3 months, the first one month period was spent for the growth of the nitrifying bacteria on the carrier bodies. The results of the experiment are shown in Table 5 below.

## Table 5

| $NH_4$-nitrogen load, kg N/m³ tank·day | Concentration of $NH_4$-nitrogen in treated water, mg/liter | Nitrification, % |
|---|---|---|
| 0.5 | 11 | 97.8 |
| 0.75 | 17 | 96.6 |
| 1.1 | 8 | 98.4 |
| 1.3 | 3.4 | 99.3 |

As is shown by the results in Table 5, the value of % nitrification was increased as the NH₄-nitrogen load was increased in the continuous running. One of the reasons therefor is presumably that the amount of the microbial bodies attached on the carrier bodies was increased along with the lapse of time. When comparison is made with the conventional process of waste water treatment by the activated sludge process in which the nitrification is usually 99 to 95% when the load of NH₄-nitrogen is 0.1 - 0.3 kg N/m³ tank·day in a continuous running, it is understood that the use of the carrier bodies according to the invention gives a great advantage that a very high value of % nitrification can be obtained even with a high nitrogen load in the nitrification runs.

**Claims**

1. A carrier body for attaching microorganisms in a cylindrical, tubular, angular or spherical form, of which the diameter and the axial length are each in the range from 3 to 100 mm, having a bulk density in the range from 0.005 to 0.4 g/cm³ and a void ratio in the range from 60 to 95% comprising three-dimensionally intercommunicating spaces formed by filaments having a diameter in the range from 10 to 1000 $\mu$m intertwisted with each other.

2. The carrier body for attaching microorganisms as claimed in claim 1 wherein the filaments are made of a synthetic resin.

3. The carrier body for attaching microorganisms as claimed in claim 2 wherein the synthetic resin is selected from the group consisting of nylon, polyester, acrylic resins, polyvinyl chloride, polyvinyl alcohol, polypropylene, polyurethane and polyvinylidene chloride.

4. The carrier body for attaching microorganisms as claimed in claim 1 wherein the intertwisted filaments are bonded together at at least a part of the contacting points therebetween by a mechanical, thermal or chemical means.

5. The carrier body for attaching microorganisms as claimed in claim 1 of which at least a part of the surface is smoothened by pressing at an elevated temperature.

6. The carrier body for attaching microorganisms as claimed in claim 1 of which at least a part of the surface is covered with a film or a net of a thermoplastic resin.

## Revendications

1. Corps support destiné à fixer des micro-organismes, de forme cylindrique, tubulaire, anguleuse ou sphérique, dont le diamètre et la longueur axiale sont tous deux compris dans l'intervalle de 3 à 100 mm, possédant une densité apparente comprise dans l'intervalle de 0,005 à 0,4 g/cm$^3$ et un rapport de vide compris dans l'intervalle de 60 à 95 %, comprenant des espaces intercommuniquant dans les trois dimensions, le corps étant formé de filaments ayant un diamètre compris dans l'intervalle de 10 à 1000 um entrelacés entre eux.

2. Corps support destiné à la fixation de micro-organismes selon la revendication 1, dans lequel les filaments sont faits d'une résine synthétique.

3. Corps support destiné à la fixation de micro-organismes selon la revendication 2, dans lequel la résine est choisie dans le groupe composé du "Nylon", du polyester, des résines acryliques, du polychlorure de vinyle, de l'alcool polyvinylique, du polypropylène, du polyuréthane et du polychlorure de vinylidène.

4. Corps support pour la fixation de micro-organismes selon la revendication 1, dans lequel les filaments entrelacés sont liés les uns aux autres, en au moins une partie des points de contact entre eux par des moyens mécaniques, thermiques ou chimiques.

5. Corps support pour la fixation de micro-organisme selon la revendication 1, dont au moins une partie de la surface est lissée par compression à chaud.

6. Corps support pour la fixation de micro-organismes selon la revendication 1, dont au moins une partie de la surface est recouverte d'un film ou filet de résine thermoplastique.

## Ansprüche

1. Trägerkörper zum Aufziehen von Mikroorganismen in einer zylindrischen, röhrenförmigen, eckigen oder kugelförmigen Form, dessen Durchmesser und axiale Länge jeweils im Bereich von 3 bis 100 mm sind, mit einer Schüttdichte im Bereich von 0.005 bis 0.4 g/cm$^3$ und einer Porenziffer im Bereich von 60 bis 95 %, der dreidimensional miteinander in Verbindung stehende Räume umfaßt, gebildet von Filamenten mit einem Durchmesser im Bereich von 10 bis 1000 $\mu$m, die miteinander verflochten sind.

2. Trägerkörper zum Aufziehen von Mikroorganismen nach Anspruch 1, wobei die Filamente aus synthetischen Harzen hergestellt sind.

3. Trägerkörper zum Aufziehen von Mikroorganismen nach Anspruch 2, wobei das synthetische Harz aus der Gruppe ausgewählt ist, die aus Nylon, Polyester, Acrylharzen, Polyvinylchlorid, Polyvinylalkohol, Polypropylen, Polyurethan, und Polyvinylidenchlorid besteht.

4. Trägerkörper zum Aufziehen von Mikroorganismen nach Anspruch 1, wobei die verflochtenen Filamente wenigstens zum Teil an den Kontaktpunkten zwischen ihnen durch mechanische, thermische oder chemische Mittel verbunden sind.

5. Trägerkörper zum Aufziehen von Mikroorganismen nach Anspruch 1, dessen Oberfläche mindestens zum Teil durch Pressen bei einer erhöhten Temperatur geglättet ist.

6. Trägerkörper zum Aufziehen von Mikroorganismen nach Anspruch 1, dessen Oberfläche mindestens zum Teil mit einem Film oder einem Netz eines thermoplastischen Harzes überzogen ist.

## FIG. 1 (a)

B

A

## FIG. 1 (b)

C

A

## FIG. 1 (c)

D

A

## FIG. 2 (a)

Air

2

1

## FIG. 2 (b)

Air

Set 1 — 1

Set 2 — 1

Set 3 — 1

Set 4 — 1

2

Air

FIG. 3

# FIG. 4

## FIG. 5

## FIG. 6

# FIG. 7

# FIG. 8

## FIG. 9

## FIG. 10

Space Velocity (m/hr)

Expansion Rate of Fluidized Bed (%)

# FIG. 11